(19) Europäisches Patentamt
European Patent Office
Office européen des brevets

(11) Veröffentlichungsnummer: **0 028 410**
**B1**

(12) **EUROPÄISCHE PATENTSCHRIFT**

(45) Veröffentlichungstag der Patentschrift: **30.09.87**

(21) Anmeldenummer: **80106714.1**

(22) Anmeldetag: **31.10.80**

(51) Int. Cl.⁴: **A 61 K 31/33,** A 61 K 31/415, A 61 K 31/44, A 61 K 31/505

(54) **Verwendung von die Thromboxan-Synthase hemmenden Verbindungen bei der Behandlung der Fettsucht und der Senkung des Insulinspiegels.**

(30) Priorität: **02.11.79 US 90850**
**02.11.79 US 90941**
**26.12.79 US 107484**

(43) Veröffentlichungstag der Anmeldung:
**13.05.81 Patentblatt 81/19**

(45) Bekanntmachung des Hinweises auf die Patenterteilung:
**30.09.87 Patentblatt 87/40**

(84) Benannte Vertragsstaaten:
**AT BE CH DE FR GB IT LI LU NL SE**

(56) Entgegenhaltungen:
**EP-A-0 003 901**
**DE-A-2 160 017**
**DE-A-2 325 300**
**US-A-3 637 731**

**CHEMICAL ABSTRACTS, Band 76, Nr. 19, 8. Mai 1972, Seite 103, Zusammenfassung Nr. 108708v, Columbus, Ohio, US, W. MALAISSE et al.: "Biochemical, pharmacological, and physiological aspects of the adenylcyclase-phosphodiesterase system in the pancreatic beta-cells"**

(73) Patentinhaber: **F. HOFFMANN-LA ROCHE & CO. Aktiengesellschaft**
**CH-4002 Basel (CH)**

(72) Erfinder: **Hamilton, James G.**
**245 High Street**
**Nutley, N.J. (US)**
Erfinder: **Lands, William E.M.**
**2651 International Drive**
**Ypsilanti, Mich. (US)**
Erfinder: **Sullivan, Ann Clare**
**48 Lakewood Avenue**
**Cedar Grove, N.J. (US)**
Erfinder: **Tobias, Lawrence D.**
**20 Douglas Road**
**Glen Ridge, N.J. (US)**
Erfinder: **Triscari, Joseph**
**98 North Fulton Street**
**Bloomfield, N.J. (US)**

(74) Vertreter: **Lederer, Franz, Dr. et al**
**Vanderwerth, Lederer & Riederer Patentanwälte**
**Lucile-Grahn-Strasse 22**
**D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 028 410 B1

(56) Entgegenhaltungen:

CHEMICAL ABSTRACTS, Band 74, Nr. 19, 10. Mai 1971, Seite 200, Zusammenfassung Nr. 96456e, Columbus, Ohio, US, P. LEFEBVRE et al.: "Effects of imidazole and two of its derivatives on insulin secretion in anesthetized dogs"

THE MERCK INDEX, 9. Ausgabe, 1976, Merck & Co., Rahway, US

Chem. Abs. 70 (1969) 113579i

**Beschreibung**

Im Rahmen der vorliegenden Erfindung wurde gefunden, dass bestimmte, die Thromboxan-Synthase hemmende Verbindungen eine den Insulinspiegel senkende und die Fettsucht hemmende Wirksamkeit aufweisen. Die den Insulinspiegel senkende Wirksamkeit dieser Thromboxan-Synthase hemmenden Verbindungen macht sie zur Behandlung von Krankheiten geeignet, die durch hohe Insulinspiegel verursacht werden. Ausser der bereits erwähnten Fettsucht sind dies Krankheiten, wie Erkrankungen der Koronararterien, der peripheren Arterien und der Gehirnarterien, sowie Insulinoma (Tumor der Insulinsekretierenden Zellen des Pankreas).

Die vorliegende Erfindung betrifft demnach die Verwendung von die Thromboxan-Synthase hemmenden Verbindungen aus der Gruppe der folgenden: Imidazole, 3-substituierte Pyridine, substituierte Indole, 4-substituierte Pyrimidine, 3-(5-Tetrazolyl)-thioxanthen-9-on-10, 10-dioxid, 1,2-Diphenyl-4-[2-(phenylsulfinyl)-äthyl]-3,5-pyrazolidindion, 1-Isopropyl-7-methyl-4-phenyl-2(1H)-chinazolinon und 7-Isopropoxy-9-oxoxanthen-2-carbonsäure zur Herstellung eines Mittels zur Behandlung der Fettsucht und der Senkung der Insulinspiegel.

Wie in CA 70, 1969, 113579j angegeben soll die Zugabe von Imidazol an aus der Ratte isoliertem Pankreasgewebe die durch exogenes Glucagon verursachte Stimulierung der Insulinsynthese unterdrücken. Den Aussagen in CA 74, 1971, 96456e ist hingegen zu entnehmen, dass man unmittelbar nach Verabreichung von Imidazol oder von seinen Derivaten Histidin oder Histamin bei anästhesierten Hunden eine beträchtliche Erhöhung der Insulinsekretion feststellen konnte und dass dieser Effekt auf die Struktur des Imidazolrings zurückzuführen ist.

Die im Rahmen der vorliegenden Erfindung verwendbaren Imidazole haben die Formel

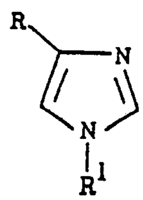

I

worin R Wasserstoff, Nitro oder $C_{2-4}$-Alkenyl und $R^1$ Wasserstoff, $C_{1-11}$-Alkyl, $C_{1-18}$-Alkanoyl, Phenyl, Naphthyl, durch $C_{1-3}$-Alkoxy, $C_{1-4}$-Alkyl, Nitro, $C_{1-18}$-Alkanoyl oder Hydroxy mono- oder disubstituiertes Phenyl, Naphthyl, Phenalkyl oder Indol-3-yl-$C_{1-4}$-alkyl.

Die im Rahmen der vorliegenden Erfindung verwendbaren 3-substituierten Pyridine haben die Formel

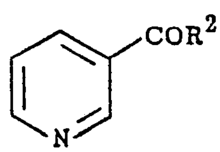

II

worin $R^2$ Wasserstoff, $C_{1-4}$-Alkyl, Phenalkyl, gegebenenfalls durch geradkettiges oder verzweigtes $C_{1-4}$-Alkyl in 2-Stellung substituiertes 3-Indolyl; Phenyl, Naphthyl oder 2-Hydroxy-1-naphthylmethylenhydrazino.

Die im Rahmen der vorliegenden Erfindung verwendbaren 4-substituierten Pyrimidine haben die Formel

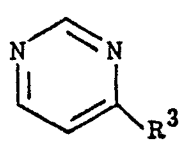

III

worin $R^3$ Phenylthio, Naphthylthio oder $C_{1-11}$-Alkoxy-oxalylmethyl ist.

Die im Rahmen der vorliegenden Erfindung verwendbaren Indole haben die Formel

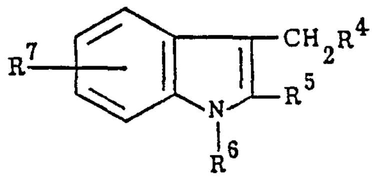

IV

worin $R^4$ Carboxy oder geradkettiges oder verzweigtes $C_{1-4}$-Alkyl, $R^5$ geradkettiges oder verzweigtes $C_{1-4}$-Alkyl, $R^6$ monochloriertes Benzoyl und $R^7$ $C_{1-4}$-Alkoxy ist.

Alkoxy bedeutet geradkettige oder verzweigte Reste, wie Methoxy, Aethoxy und Isopropoxy. Alkenyl bedeutet geradkettige oder verzweigte, eine Doppelbindung enthaltende, einwertige aliphatische Reste. Beispiele von $C_{1-18}$-Alkanoylresten sind Acetyl, Propionyl und Butyryl. In einem Phenylrest ist der Hydroxysubstituent vorzugsweise in para-Stellung.

Beispiele von die Thromboxan-Synthase hemmenden Verbindungen sind:

4-Imidazolo-phenol,
3-Imidazolmethyl-indol
1-(2-Isopropylphenyl)-imidazol
2-Isopropyl-3-nicotinoyl-indol
1-(4-Imidazolo-phenyl)-äthanon
3-Imidazolo-phenol
1-(4-Isopropoxyphenyl)-imidazol
1-(4-Aethoxyphenyl)-imidazol
1-Butylimidazol
1-Nonylimidazol
1-Triphenylmethyl-imidazol
1-Benzylimidazol
1-[(2-Chlorphenyl)-diphenylmethyl]-imidazol
1-(4-Methoxyphenyl)-imidazol
4-Phenylpyrimidin
1-Nicotinoyl-2-(2-hydroxy-1-napthylmethylen)-hydrazin Imidazol
1-(4-Nitrophenyl)imidazol
1-(2,4-Dinitrophenyl)-imidazol
1-[2-(2,4-Dichlorphenyl)-2-[(2,4-dichlorphenyl)-methoxy]-äthyl]-imidazol
Methyl-4-imidazol-acrylat
2-Methyl-1,2-di-3-pyridyl-1-propanon-bis-tartrat
[1-(4-Chlorbenzoyl)-5-methoxy-2-methyl-3-indolyl]-essigsäure
4-(Aethoxy-oxalylmethyl)-pyrimidin
3-(5-Tetrazolyl)-thioxanthen-9-on-10,10-dioxid
1,2-Diphenyl-4-[2-(phenylsulfinyl)-äthyl]-3,5-pyrazolidindion
1-Acetylimidazol
1-Methylimidazol
1-Aethylimidazol
4-Nitroimidazol
1-Isopropyl-7-methyl-4-phenyl-2[1H]-chinazolinon
7-Isopropoxy-9-oxoxanthen-2-carbonsäure

Eine Anzahl von die Thromboxan-Synthase hemmenden Verbindungen wurden auf ihre den Insulinspiegel senkende Wirksamkeit in vitro getestet. Diese Verbindungen wurden ferner auf ihre die Fettsucht hemmende Wirksamkeit in vivo getestet.

Im Rahmen der vorliegenden Erfindung bevorzugte Verbindungen sind Imidazole der Formel I, worin R Wasserstoff und $R^1$ $C_{1-9}$-Alkyl, Indol-3-ylmethyl oder p-Hydroxyphenyl sind; und Pyridine der Formel II, worin $R^2$ in 2-Stellung durch geradkettiges oder verzweigtes $C_{1-4}$-Alkyl substituiertes 3-Indolyl ist. Die folgenden Verbindungen sind besonders bevorzugt:

4-Imidazolophenol, 3-Imidazolomethyl-indol, 1-(2-Isopropylphenyl)-imidazol, 1-(4-Imidazolophenyl)-äthanon, 3-Imidazolophenol, 1-(4-Isopropoxyphenyl)-imidazol, 1-(4-Aethoxyphenyl)-imidazol, 1-Nonylimidazol, 1-Butylimidazol, 1-Benzylimidazol, 1-(4-Methoxyphenyl)-imidazol, 1-(2,4-Dinitrophenyl)-imidazol, 1-(4-Nitrophenyl)-imidazol, Imidazol, 1-Acetylimidazol, 1-Aethylimidazol, 1-Methylimidazol, 4-Nitroimidazol oder 2-Isopropyl-3-nicotinoyl-indol.

Im Rahmen der vorliegenden Erfindung können die Thromboxan-Synthase hemmende Verbindungen in Form von pharmazeutisch annehmbaren nicht-toxischen Salzen, vorzugsweise Hydrochloride, Nitrate, Sulfate und Tartrate, verwendet werden.

Eine geeignete pharmazeutische Dosierungsmenge kann 0,1 bis 200, vorzugsweise 0,1 bis 50, besonders bevorzugt 0,1 bis 10 mg/kg/Tag sein.

Im Rahmen der vorliegenden Erfindung können die Thromboxan-Synthase hemmende Verbindungen mit herkömmlichen organischen oder anorganischen Trägermaterialien, wie Wasser, Gelatine, Gummi,

Lactose, Stärke, Magnesiumstearat, Talk, pflanzliche Oele, Polyalkylenglykol und Vaseline® verabreicht werden. Auf diese Weise hergestellte pharmazeutische Präparate können in Einzeldosierungsformen vorliegen und andere therapeutisch wertvolle Stoffe oder herkömmliche Hilfsstoffe, wie Konservierungs-, Stabilisierungs-, Netz- oder Emulgiermittel und Puffer enthalten. Diese Präparate können in fester Form, z.B. als Tabletten, Kapseln und Dragées, in halbfester Form, z.B. als Salben, in flüssiger Form, z.B. als Lösungen, Suspensionen oder Emulsionen, oder in anderen herkömmlichen Formen, wie Trockenampullen und Suppositorien, vorliegen. Sie können herkömmlichen Behandlungsmethoden, wie Sterilisation, unterworfen werden.

Die folgenden Beispiele veranschaulichen die den Insulinspiegel senkende und die Fettsucht hemmende Wirksamkeit der Thromboxan-Synthase-Inhibitoren.

Beispiel 1

Bestimmung der Insulin-Sekretion im isolieren Ratten-Pankreas.

Anästhesierten weiblichen Charles River Ratten wurde der Pankreas entnommen und mit einer Perfusions-Apparatur verbunden. Die Perfusionslösung enthielt 133 mN NaCl, 4,4 mM KCl, 2,5 mM $MgSO_4$, 2,4 mM Ca-gluconat, 1,5 mM $KH_2PO_4$, 29,4 mM $NaHCO_3$, 4% Dextran, 0,2% Rinderserumalbumin und Glucose wie weiter unten angegeben. Die Perfusionsgeschwindigkeit wurde bei 4 ml/Min. konstant gehalten.

Die Perfusion wurde wie folgt durchgefürt:

10 Minuten mit 5,6 mM Glucose.
30 Minuten mit 16,7 mM Glucose (interne Kontrolle).
10 Minuten mit 5,6 mM Glucose.
30 Minuten mit 16,7 mM Glucose und Testsubstanz.
10 Minuten mit 16,7 mM Glucose.
10 Minuten mit 5,6 mM Glucose.
5 Minuten mit 20 mM Arginin (Kontrolle der Pankreaslebensfähigkeit.

Die Testverbindungen wurden in 90% Aethanol gelöst und die Lösung wurde auf die erwünschte Konzentration verdünnt. Der Perfusionsausfluss wurde jede Minute oder alle 4 Minuten gesammelt und in den gesammelten Fraktionen die Insulin-Sekretion mittels Radioimmunoassay bestimmt. Zur Bestimmung der hemmenden Wirksamkeit der Testverbindungen wurde ein Hemmungsindex berechnet. Dieser Index ist das Verhältnis zwischen dem Insulin-Sekretionswert der Kontrollperfusion und dem mit der Testverbindung erhaltenen Wert. Wie aus Tabelle I hervorgeht, bewirken die die Thromboxan-Synthase hemmenden Verbindungen der vorliegenden Erfindung eine beträchtliche Hemmung der Insulin-Sekretion.

TABELLE I

| Verbindung | Insulin-Sekretion Konzentration µM | Insulin-Sekretion Hemmung Index |
|---|---|---|
| Kontrolle | | 1 |
| Imidazol | 100<br>1000 | 2<br>3 |
| 1-(Triphenylmethyl)-imidazol | 10 | 2 |
| 1-(4-Imidazolophenyl)-äthanon | 100 | 2 |
| 4-Imidazolo-phenol | 10 | 2 |
| 3-(Imidazolomethyl)-indol | 10<br>50<br>100 | 1<br>13<br>32 |
| 1-(4-Isopropoxyphenyl)-imidazol | 100 | 4 |
| 2-Isopropyl-3-nicotinoyl-indol | 100 | 5 |
| 1-Benzylimidazol | 1000 | 3 |
| 1-[(2-Chlorphenyl)-diphenylmethyl]-imidazol | 10 | 3 |
| 3-Imidazolo-phenol | 1000 | 2 |
| 1-(2-Isopropylphenyl)-imidazol | 10<br>1000 | 2<br>25 |
| 1-Butylimidazol | 1000 | 5 |

Beispiel 2

Bestimmung der Wirksamkeit gegen Fettsucht in vivo.

Magere und fette männliche und weibliche Zuckerratten wurden 1 bis 3 Wochen vor Versuchsbeginn ad libitum gefüttert. Dann wurden sie mit glucosereicher fettarmer (1% Maisöl) Diät (G—70:1% CO) gefüttert. Nach dieser 1- bis 4-wöchentlichen Periode wurden die Ratten in Gruppen von 4 bis 7 Tieren aufgeteilt. Der Nahrungsverbrauch und das Körpergewicht wurden täglich oder zweimal in der Woche gemessen. Für jede Tiergruppe wurde die Wirksubstanz in einer dem Nahrungsverbrauch pro kg Körpergewicht entsprechenden Menge verabreicht. Die Gewichtsangaben entsprechen der Differenz zwischen den Gewichtszunahmen der Kontrolltiere und der behandelten Tiere. Der Nahrungsverbrauch und der Insulinspiegel wurden als % der Kontrollen angegeben. Wie aus Tabelle II ersichtlich konnte die Gewichtszunahme durch Verabreichung der Verbindungen der vorliegenden Erfindung beträchtlich gesenkt werden.

TABELLE II

| Verbindung | Dosis[a] (mg/kg) | Körpergewichtzunahme (g) | Nahrungsverbrauch (% der Kontrolle) | Insulin (% der Kontrolle) |
|---|---|---|---|---|
| Kontrolle | | | 100 ± 3 | 100 ± 15 |
| 1-(4-Imidazolophenyl)-äthanon | 60 | −25* | 82 ± 3* | 66 ± 16 |
| | 87 | −32* | 84 ± 1* | 32 ± 5* |
| | 113 | −74* | 74 ± 1* | 24 ± 1* |
| 3-(Imidazolomethyl)-indol | 18 | −24* | 81 ± 1* | 72 ± 9* |
| | 55 | −68* | 63 ± 4* | 12 ± 2* |
| 1-(4-Isopropoxyphenyl)-imidazol | 82 | −18* | 80 ± 2* | 94 ± 21 |
| 2-Isopropyl-3-nicotinoyl-indol | 94 | −17* | 89 ± 4 | |
| 1-(2-Isopropylphenyl)-imidazol | 42 | −23* | 91 ± 5 | 67 ± 17 |
| | 77 | −84* | 73 ± 3* | 35 ± 4 |
| 1-Nonylimidazol | 100 | −31* | 86 ± 1* | 55 ± 2 |
| 1-Butylimidazol | 86 | −61* | 83 ± 1* | 45 ± 2 |

[a] mittlere Behandlungsdauer: 16—18 Tage

* erheblich verschieden von der Kontrolle ($P \leq 0{,}05$)

Bei einer Tiergruppe wurden die Kadaver untersucht. Nach der weiter oben beschriebenen Behandlung wurden die Ratten getötet, die Leber und das Blut entfernt, die Kadaver gewogen, mit alkoholischer Kaliumhydroxydlösung verseift, angesäuert und mit Petroläther extrahiert. Die Petrolätherextrakte wurden zur Trockene eingedampft und gewogen. Ein Teil des verseiften Kadaverextraktes wurde neutralisiert und dessen Stickstoffinhalt ermittelt. In Tabelle III ist der Lipid- und der Protein-Inhalt der Kadaver in g sowie in % des Kadavergewichts angegeben. Durch Verabreichung der die Thromboxan-Synthase hemmenden Verbingdungen der vorliegenden Erfindung konnte das Körpergewicht durch beträchtliche Körperfettabnahme gesenkt werden. Wie erwünscht, blieb dabei der Proteinspiegel unverändert.

TABELLE III

Einfluss von 1-(4-Imidazolophenyl)-äthanon auf das Kadavergewicht und die Kadaver-Lipid- und Kadaver-Protein-Spiegel bei männlichen Zuckerratten

| Behandlung | Genotyp | Kadavergewicht g | Kadaver-Lipide Gesamtgewicht (g) | Kadaver-Lipide % des Kadavergewichts | Kadaver-Proteine Gesamtgewicht (g) | Kadaver-Proteine % des Kadavergewichts |
|---|---|---|---|---|---|---|
| Kontrolle | mager | $428 \pm 9$ (100)[b] | $57 \pm 2$ (100) | $13 \pm 0$ (100) | $81 \pm 6$ (100) | $19 \pm 1$ (100) |
| 1-(4-Imidazolophenyl)-äthanon (98 mg)[a] | mager | $361 \pm 27$* (84) | $29 \pm 5$* (50) | $8 \pm 1$* (60) | $75 \pm 5$ (87) | $21 \pm 2$ (111) |
| Kontrolle | fett-leibig | $556 \pm 22$ (100) | $263 \pm 13$ (100) | $47 \pm 1$ (100) | $74 \pm 3$ (100) | $13 \pm 1$ (100) |
| 1-(4-Imidazolophenyl)-äthanon (87 mg)[a] | fett-leibig | $460 \pm 28$* (83) | $225 \pm 6$ (86) | $49 \pm 1$ (104) | $81 \pm 4$ (109) | $18 \pm 2$ (131) |

[a] tägliche 1-(4-Imidazolophenyl)-äthanon-Einnahme pro kg/Körpergewicht
[b] Prozente der Kontrolle
* $P \leq 0.05$

**Patentansprüche**

1. Verwendung von die Thromboxan-Synthase hemmenden Verbindungen der Formeln

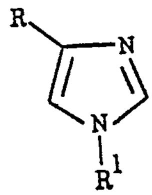 I 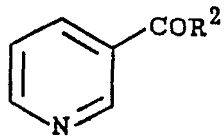 II 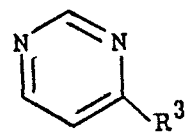 III

oder 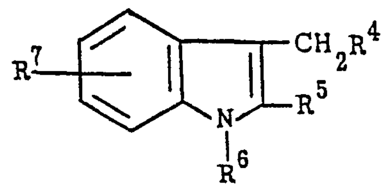 IV

worin

R Wasserstoff, Nitro oder $C_{2-4}$-Alkenyl und $R^1$ Wasserstoff, $C_{1-11}$-Alkyl, $C_{1-18}$-Alkanoyl, Phenyl, Naphthyl, durch $C_{1-3}$-Alkoxy, $C_{1-4}$-Alkyl, Nitro, $C_{1-18}$-Alkanoyl oder Hydroxy mono- oder di-substituiertes Phenyl, Naphthyl, Phenalkyl oder Indol-3-yl-$C_{1-4}$-alkyl;

$R^2$ Wasserstoff, $C_{1-4}$-Alkyl, Phenalkyl, gegebenenfalls durch geradkettiges oder verzweigtes $C_{1-4}$-Alkyl, in 2-Stellung substituiertes 3-Indolyl; Phenyl, Naphthyl oder 2-Hydroxy-1-naphthylmethylenhydrazino;

$R^3$ Phenylthio, Naphthylthio oder $C_{1-11}$-Alkyl-oxalylmethyl;

$R^4$ Carboxy oder geradkettiges oder verzweigtes $C_{1-4}$-Alkyl, $R^5$ geradkettiges oder verzweigtes $C_{1-4}$-Alkyl, $R^6$ monochloriertes Benzoyl und $R^7$ $C_{1-4}$-Alkoxy, ist; eines Salzes einer Verbindung der Formel I, II, oder III mit einer pharmazeutisch anwendbaren Säure, oder von die Thromboxan-Synthase hemmenden Verbindungen aus der Gruppe der folgenden: 3-(5-Tetrazolyl)-thioxanthen-9-on-10,10-dioxyd, 1,2-Diphenyl-4-[2-(phenyl-sulfinyl)-äthyl]-3,5-pyrazolidindion, 1-Isopropyl-7-methyl-4-phenyl-2[1H]-chinazolinon und 7-Isopropoxy-9-oxo-xanthen-2-carbonsäure zur Herstellung eines Mittels zur Behandlung der Fettsucht und der Senkung des Insulinspiegels.

2. Verwendung nach Anspruch 1, dadurch gekennzeichnet, dass man eine Verbindung aus der Gruppe der folgenden verwendt: 4-Imidazolophenol, 3-Imidazolomethyl-indol, 1-(2-Isoproplyphenyl)-imidazol, 1-(4-Imidazolophenyl)-äthanon, 3-Imidazolophenol, 1-(4-Isopropoxyphenyl)-imidazol, 1-(4-Aethoxyphenyl)-imidazol, 1-Nonylimidazol, 1-Butylimidazol, 1-Benzylimidazol, 1-(4-Methoxyphenyl)-imidazol, 1-(2,4-Dinitrophenyl)-imidazol, 1-(4-Nitrophenyl)-imidazol, Imidazol, 1-Acetylimidazol, 1-Aethylimidazol, 1-Methylimidazol, 4-Nitroimidazol oder 2-Isopropyl-3-nicotinoyl-indol.

**Revendications**

1. Utilisation de composés inhibiteurs de la thromboxane-synthétase, de formules

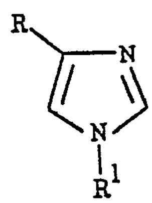 I 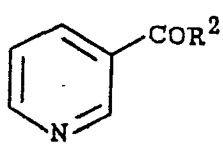 II 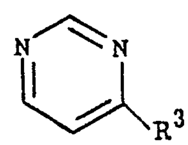 III

ou 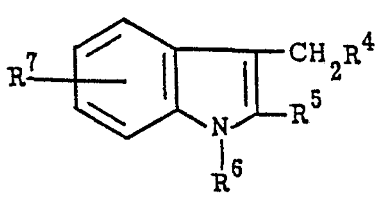 IV

dans lesquelles

R représente l'hydrogène, un groupe nitro ou alcényle en $C_2$—$C_4$ et $R^1$ représente l'hydrogène, un groupe alkyle en $C_1$—$C_{11}$, alcanoyle en $C_1$—$C_{18}$, phényle, naphtyle, phényle mono- ou di-substitué par des groupes alcoxy en $C_1$—$C_3$, alkyle en $C_1$—$C_4$, nitro, alcanoyle en $C_1$—$C_{18}$ ou hydroxy, naphtyle, phénalkyle ou indole-3-yl-alkyle en $C_1$—$C_4$;

$R^2$ représente l'hydrogène, un groupe alkyle en $C_1$—$C_4$, phénalkyle, 3-indolyle éventuellement

substitué en position 2 par un groupe alkyle à chaîne droite ou ramifiée en $C_1$—$C_4$; phényle, naphtyle ou 2-hydroxy-1-naphtyl-méthylène-hydrazino;

$R^3$ représente un groupe phénylthio, naphtylthio ou (alcoxy en $C_1$—$C_{11}$)-oxalylméthyle;

$R^4$ représente un groupe carboxy ou alkyle à chaîne droite ou ramifiée en $C_1$—$C_4$, $R^5$ représente un groupe alkyle à chaîne droite ou ramifiée en $C_1$—$C_4$, $R^6$ représente un groupe benzoyle monochloré et $R^7$ un groupe alcoxy en $C_1$—$C_4$; d'un sel d'un composé de formule I, II ou III et d'un acide acceptable pour l'usage pharmaceutique, ou de composés inhibiteurs de la thromboxane-synthétase, pris dans le groupe formé par les suivants: 10,10-dioxyde de la 3-(5-tétrazolyl)-thioxanthène-9-one, 1,2-diphényl-4-[2-phényl-sulfinyl)-éthyl]-3,5-pyrazolidine-dione, 1-isopropyl-7-méthyl-4-phényl-2[1H]-quinazoinone et acide 7-isopropoxy-9-oxo-xanthène-2-carboxylique, pour la préparation d'un agent pour le traitement de l'obésité et pour diminuer les sécrétions d'insuline.

2. Utilisation selon la rev. 1, caractérisée en ce que l'on utilise un composé du groupe des suivants: 4-imidazolophénol, 3-imidazolométhyl-indole, 1-(2-isopropylphényl)-imidazole, 1-(4-imidazolophényl)-éthanone, 3-imidazolo-phénol, 1-(4-isopropoxyphényl)-imidazole, 1-(4-éthoxyphényl)-imidazole, 1-nonylimidazole, 1-butylimidazole, 1-benzylimidazole, 1-(4-méthoxyphényl)-imidazole, 1-(2,4-dinitrophényl)-imidazole, 1-(4-nitrophényl)-imidazole, imidazole, 1-acétylimidazole, 1-éthylimidazole, 1-méthylimidazole, 4-nitroimidazole ou 2-isopropyl-3-nicotinoyl-indole.

## Claims

1. The use of the thromboxane-synthase inhibiting compounds of the formulae

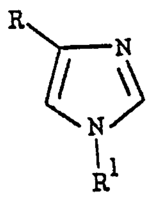

I

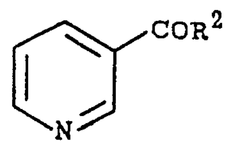

II

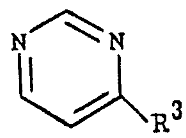

III

or

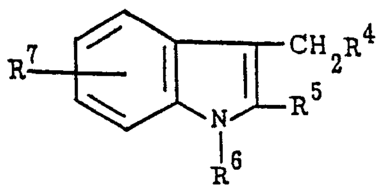

IV

wherein

R is hydrogen, nitro or $C_{2-4}$-alkenyl, and $R^1$ is hydrogen, $C_{1-11}$-Alkyl, $C_{1-18}$-alkanoyl, phenyl, naphthyl, phenyl, naphthyl, phenalkyl or indol-3-yl-$C_{1-4}$-alkyl, mono- or disubstituted by $C_{1-3}$-alkoxy, $C_{1-4}$-alkyl, nitro, $C_{1-18}$-alkanoyl or hydroxy;

$R^2$ is hydrogen, $C_{1-4}$-alkyl, phenalkyl, 3-indolyl optionally substituted in the 2-position by straight-chain or branched $C_{1-4}$-alkyl; phenyl, naphthyl or 2-hydroxy-1-naphthylmethylenehydrazino;

$R^3$ is phenylthio, naphthylthio or $C_{1-11}$-alkoxy-oxalylmethyl;

$R^4$ is carboxy or straight-chain or branched $C_{1-4}$-alkyl, $R^5$ is straight-chain or branched $C_{1-4}$-alkyl, $R^6$ is monochlorinated benzoyl and $R^7$ is $C_{1-4}$-alkoxy; a salt of a compound of formula I, II or III with a pharmaceutically usable acid, or of the thromboxane-synthase inhibiting compounds from the following group: 3-(5-tetrazolyl)-thioxanthen-9-one, 10,10-dioxide, 1,2-diphenyl-4-[2-(phenyl-sulphinyl)-ethyl]-3,5-pyrazolidinedione, 1-isopropyl-7-methyl-4-phenyl-2[1H]-quinazolinone and 7-isopropoxy-9-oxo-xanthene-2-carboxylic acid for the manufacture of a medicament for the treatment of obesity and of the lowering of the insulin level.

2. The use according to claim 1, characterized in that a compound from the following group is used: 4-imidazolophenol, 3-imidazolomethyl-indole, 1-(2-isopropylphenyl)-imidazole, 1-(4-imidazolophenyl)-ethanone, 3-imidazolophenol, 1-(4-isopropoxyphenyl)-imidazole, 1-(4-ethoxyphenyl)-imidazole, 1-nonylimidazole, 1-butylimidazole, 1-benzylimidazole, 1-(4-methoxyphenyl)-imidazole, 1-(2,4-dinitrophenyl)-imidazole, 1-(4-nitrophenyl)-imidazole, imidazole, 1-acetylimidazole, 1-ethylimidazole, 1-methylimidazole, 4-nitroimidazole or 2-isopropyl-3-nicotinoyl-indole.